# EUROPEAN PATENT SPECIFICATION

(11) **EP 0 611 162 B1**
(45) Date of publication and mention of the grant of the patent: **18.11.1998**
(21) Application number: 94300998.5
(22) Date of filing: 11.02.1994
(51) Int. Cl.: A01N 59/00

(54) **Chlorine dioxide generating compositions**
Chlordioxid erzeugende Zusammensetzungen
Compositions de génération de dioxyde de chlore

(30) Priority: 12.02.1993 US 17657
(43) Date of publication of application: 17.08.1994
(73) Proprietor: SOUTHWEST RESEARCH INSTITUTE, San Antonio Bexar County Texas (US)
(72) Inventor: Wellinghoff, Stephen T., San Antonio Bexar County Texas 78250 (US)
(74) Representative: W.P. THOMPSON & CO.

(56) References cited:
- EP-A- 0 287 074
- GB-A- 2 151 138
- US-A- 4 547 381
- US-A- 4 585 482
- DATABASE WPI Week 8303, Derwent Publications Ltd., London, GB; AN 83-06209K [03] & JP-A-57 198 775 (NASA) 6 December 1982
- DATABASE WPI Week 9229, Derwent Publications Ltd., London, GB; AN 92-239876 [29] & JP-A-4 164 005 (TAKASUGI SEIYAKU) 9 June 1992

## Description

### TECHNICAL FIELD OF THE INVENTION

The present invention relates generally to a composition for reducing bacterial and fungal growth and repelling insects, and more particularly to an ionomeric composition having biocidal or insect repelling releasing agent and method for making same.

### BACKGROUND OF THE INVENTION

Chlorine Dioxide ("ClO₂") has been incorporated in polymer [JP 63,296,758, NOK Corp, 1988; CA 111 P120181s] and ceramic beads [JP 63,274,434, Enkler Busin.K.K., 1988; CA 111 P11975h], and in a calcium silicate wrapped in non-woven cloth [JP 57,168,977, Enkler Busin.K.K., 1982; CA 98 P77552] to be used as a deodorant. Food preservation is an application where a gel form is preferred [JP-A-57022102, Diamaru Kogyo Kaishi, Ltd., 1982; CA 97 22409]. Chlorine dioxide generating gels have also been used as topical applications for disinfection [A.J. Kenyon, S.G. Hamilton and D.M. Douglas, Am.J.Vet.Res., 45(5), 1101 (1986)].

Most attempts to generate chlorine dioxide involve acidification (and or chlorination) of an aqueous solution of chlorite, acidification (and/or reduction) of chlorates ["Comprehensive Inorganic Chemistry, The Halogens", Vol 3, R.C.Brasted, D. Van Nostrand Co., New York, 1954, p134-142] or reduction of a chlorite solution with aldehydes [US 2,323,594, Mathieson Alkali Works, 1943]. Typically the preparation involves mixing component A (gel with suspended NaClO₂) with component B (gel with lactic acid) immediately prior to use. α-hydroxy carboxylic acids (citric, tartaric, malic, glycolic) are especially useful for generating ClO₂ by the reaction: ${\text{H}}^{\text{+}} {\text{+ NaClO}}_{\text{2}} {\text{→ HClO}}_{\text{2}} {\text{+ Na}}^{\text{+}}$${\text{5HClO}}_{\text{2}} {\text{→ 4ClO}}_{\text{2}} {\text{+ HCl + 2H}}_{\text{2}} \text{O}$[Ger.Offen. 2,817,942, H.Allinger, 1979; WO-A-8808823, J. Mason, 1988, CA 110 P98205h]. Alternatively, NaClO₂ and lactic acid have been separately encapsulated in polyvinyl alcohol and then mixed with water to generate ClO₂ [Can. 959,238, Chemical Generators, 1974]. Formulations involving sulfonic acids are also available [Europ. Pat. Appl. EP 287,074, Alcide Corp., 1987; CA 111 P45299f].

Another method for the production of chlorine dioxide employs acetic anhydride in the reaction: ${\text{2NaClO}}_{\text{2}} {\text{+ (MeCO)}}_{\text{2}} {\text{O + H}}_{\text{2}} {\text{O → ClO}}_{\text{2}} {\text{+ NaCl + MeCOOH + H}}^{\text{+}}$${\text{+MeCOONa + O}}_{\text{2}} \text{(The unbalanced proton takes part in reaction}$ Interestingly prehydrolysis of the anhydride to acetic acid prior to the addition of chlorite produces no chlorine dioxide. Direct reaction of chlorite with anhydride in the presence of water is obviously important in the reaction [W. Masschelein, I and EC Prod. Res. and Dev., 6(2), 137 (1967)].

This work suggests the possibility of storing chlorite in a complex with an acid anhydride in a stable form until reaction with moisture liberates the chlorine dioxide [US 2,482,134, R.Aston, 1949). F o o d packaging with the incorporated disinfectant, chlorine dioxide, brings up an important public health question pertinent to this practice: Does chronic ingestion of residual levels of disinfectants result in a significant genetic or carcinogenetic hazard to the human population? It is clear from published pharmacokinetic studies aided by incorporation of a chlorine radioisotope (³⁶Cl) that skin patch (Alcide gel) administration of chlorine dioxide and chlorite result in prolonged systemic residence of chlorine containing residues [J. Scatina, M. S. Abdel-Rahman, S.E. Gerges, Y. Khan and O. Gona, Fund.Appl.Tox., 4, 479 (1984)].

Meier et.al. published a report on the effect of subchronic and acute oral administration of chlorine, chlorine dioxide, sodium chlorite, and sodium chlorate on the induction of chromosomal aberrations and sperm-head abnormalities in mice [J.R. Meier, R.J. Bull, J.A. Stober and M.C. Cimino, Environ. Mutagenesis, 7, 201 (1985)]. Only the highly reactive hypochlorite resulted in a weak positive effect for mutagenic potential. The disinfectants failed to induce any chromosomal aberrations or increased numbers of micronuclei in the bone marrow of mice. Other hazards which have been associated with chlorine dioxide but were not incorporated in the published study include hemolytic anemia and hypothyroidism. One of the reasons for the relatively innocuous effect of ClO₂ is its inability to produce halomethanes, unlike hypochlorite and chlorine [R. Vilagines et al., Proc. AWWA Disinfect. Semin. 1977, paper 3, 24pp; CA 93 173513f].

U.S. Patent No. 4,585,482 describes the gradual hydrolysis of alternating poly (vinyl methyl ether-maleic anhydride) or poly (lactic-glycolic acid) to generate acid which can release chlorine dioxide from sodium chlorite. In the process a solution process can be used to encapsulate a polyalcohol humectant and water with the polyanhydride or polyacid in a nylon coating. After sodium chlorite is permitted to diffuse into the capsule through the nylon wall, an impermeable polystyrene layer is coacervated around the nylon capsule. The capsules can be coated onto surfaces to release chlorine dioxide and provide biocidal action for several days to months.

Despite the many advantages of the '482 patent, there are some limitations. First, a large number of processing steps involving numerous chemical reactions and physical processes with disposal problems are required. Second, the chemistry is limited to batch processes. Third, a clear film cannot be produced. Finally, chlorine dioxide release starts immediately.

### SUMMARY OF THE INVENTION

The object of the present invention is to improve over the prior art by a method which is continuous, safer and has minimal number of preparation steps.

The more specific objects of the invention are: to provide a low cost, chlorite containing oligomer or polymer that can be coated onto surfaces as a film prior to chlorine dioxide release. In addition, the invention provides a chlorite loaded film which will release chlorine dioxide over an extended period of time by exposure to moisture and both disinfect and repel insects from the surface onto which it is coated.

In the presence of water the local pH in the polymer film is reduced, releasing the chlorine dioxide.

According to the present invention there is provided a composition for disinfection, reducing bacterial and fungal growth, and repelling insects comprising:
a first phase containing a chlorite salt dissolved in a hydrogen-bonded matrix; and
an apolar second phase containing an anhydride and a diluent, the first and second phases being in intimate contact and incompatible with one another, and the first phase being capable of releasing chlorine dioxide upon hydrolysis of the anhydride.

There is also provided, in accordance with the invention a composition for disinfection, reducing bacterial and fungal growth, and repelling insects comprising:
a first phase containing a chlorite salt dissolved in a hydrogen-bonded matrix selected from an amide, an alcohol, or a polymerizable hydrogen-bonded epoxide or amine; and
an apolar second phase containing an anhydride, the first and second phases being in intimate contact and incompatible with one another and the first phase being capable of releasing chlorine dioxide upon hydrolysis of the anhydride.

The invention further provides a process for preparing a composition comprising:
dissolving a chlorite salt in a first material containing a hydrogen-bonded matrix to form a first phase; and
then mixing the first phase with an apolar phase containing an anhydride and a diluent, the first and second phases being incompatible with each other, the first phase being capable of releasing chlorine dioxide upon hydrolysis of the anhydride.
It additionally provides a process for preparing a composition comprising:
dissolving a chlorite salt in a first material containing a hydrogen-bonded matrix to form a first phase, the hydrogen-bonded matrix being selected from an amide, an alcohol, or a polymerizable hydrogen-bonded epoxide or amine; and
then mixing the first phase with an apolar second phase containing an anhydride, the first and second phases being incompatible with each other, the first phase being capable of releasing chlorine dioxide upon hydrolysis of the anhydride.

The present invention disclosed herein thus comprises a method for making a composition which liberates a biocidal and/or insect repelling agent comprising the steps of dissolving a chlorite salt into a hydrogen bonded matrix to create a chlorite containing phase. The chlorite containing phase is then mixed with an incompatible phase having a hydrolyzable anhydride component therein to create a mixture having an ionomeric polymer. By exposing this mixture to the atmospheric moisture the ionomeric polymer liberates a chlorine dioxide compound to in effect act as a biocidal agent or an insect repelling agent.

In addition, the present invention discloses a composition which liberates a biocidal and/or insect repelling agent when exposed to a water source in accordance with the method of making as described above.

### DETAILED DESCRIPTION

The preferred constituents of the apolar phase are styrene - maleic anhydride copolymers and their grafts with olefins. The monomers are cheap, commercially available and will form charge transfer complex that will polymerize thermally in the presence of small amounts of oxygen. Maleic anhydride contents between 0-50 mole% are available. At maleic anhydride concentrations below 33% the copolymers are soluble in apolar aromatic plasticizers.

In a preferred process an equimolar styrene-maleic anhydride monomer mix at 60°C is added to a melt of plasticized, low molecular weight olefin such as, atactic polypropylene or other olefin in a Brabender Plasticorder equipped with a twin screw extruder. The styrene maleic-anhydride will spontaneously polymerize to a macroradical which will graft to the base polymer [N. Gaylord, ACS Adv.Chem.Ser., 129, 209 (1973); Z. Kopicova, J. Nemic and M. Protiva, Coll. Czechoslov.Chem. Commun. 41, 459 (1976)].

An alternative method to produce the desired anhydride substituted polymer is to copolymerize methacrylic anhydride with hydrophobic, aliphatic or aromatic vinyl monomers. Linear polymers that incorporate anhydride in the backbone generally require that the anhydride be included in mole percentages less than 6% [J.C.H. Hwa and L. Miller, J. Poly. Sci., 55, 197 (1961)].

The apolar phase may also contain components which may be induced to crosslink after film coating in order to stabilize the mechanical properties of the coating. These components include mixtures of hydrophobic epoxides and amines.

The gradual hydrolysis of the anhydride component in the presence of atmospheric moisture will generate acid functionality and ionomer formation. This will promote interphase compatiblization between the chlorite containing hydrogen bonded phase and the apolar phase containing the anhydride.

The preferred constituents of the hydrogen bonded phase include sodium chlorites or other chlorites where the cation may be an alkali, alkaline earth metal or transition metal ion or a protonated primary, secondary, tertiary amine or quaternary amine. The hydrogen bonded phase which solubilizes the chlorite can be monomeric or polymeric amides (e.g. formamide, isopropylacrylamide-acrylamide mixture) or monomeric or polymeric alcohols (e.g. methanol, 2-(2-ethoxyethanol). The amides are especially preferred since the amide functionality does not react with the anhydride below 150°C. In addition, chlorites, such as sodium chlorite remain stable in amides, such as formamide, up to 100°C.

The hydrogen bonded phase may also contain components which may be induced to polymerize after film coating in order to stabilize the mechanical properties of the coating. These components include hydrogen bonded epoxides and amines.

The final coating composition is made by mixing the hydrophobic and hydrogen bonded phases to the desired dispersion at temperatures below the decomposition temperature of the chlorite, preferably at temperatures less than 60°C. The coating is then applied as a hot melt to the substrate and then is induced to solidify by cooling to room temperature.

If both phases are mixed for short times at high viscosity little reaction of the anhydride with the chlorite occurs. However, if the dispersion is very fine, the phase compatibility very high (similar solubility parameters for the apolar phase and hydrogen bonded phase) and the viscosity of the phase is low extensive production of chlorine dioxide is observed even in dry films.

Exposure of the solid, unreacted film to water will hydrolyze the anhydride groups, compatiblize the phases and induce chlorine dioxide release. Thus chlorine dioxide release can be controlled by phase dispersion, phase viscosity, and exposure to atmospheric moisture.

The present invention is further illustrated by the following example.

A 7 wt% solution of sodium chlorite was made in 33wt% formamide:33wt% acrylamide:33wt% isopropylacrylamide (hydrogen bonded phase). Next a 40% solution of a 33mole%maleic anhydride:66%moles styrene copolymer solution in ethylbenzene plasticizer (apolar phase) was vortex mixed with the hydrogen bonded, chlorite containing phase. The resultant white blend of the two disperse phases started a sustained release of chlorine dioxide in the absence of added water within five minutes at room temperature due to interphase diffusion and reaction.

The release rate could be slowed by cooling the mixture to 0°C or by increasing the viscosity of the phases. Water was observed to hydrolyze the maleic anhydride to maleic acid and increase the rate of chlorine dioxide release.

While the preferred embodiments of the invention and their advantages have been disclosed in the above detailed description, the invention is not limited thereto but only by the scope of the appended claims.

### The following paragraphs summarise some important features of the invention:

The method for making the composition which liberates a repelling or biocidal agent comprises the steps of:
dissolving a chlorite salt of a cation into hydrogen bonded matrices to create an amine containing phase; and
mixing said amine containing phase with an incompatible phase having a hydrolyzable anhydride component therein to create a mixture having an ionomeric polymer; and
exposing said mixture to atmospheric moisture such that said ionomeric polymer releases chlorine dioxide when exposed to said moisture in order to liberate a repelling or biocidal agent.

The chlorite cation may comprise an alkali, alkaline earth, transition metal, protonated primary amine, protonated secondary amine, protonated secondary amine, protonated tertiary amine, quaternary monomeric amine, quaternary polymeric amine or a mixture thereof.

The method may further include the step of selectively forming a film of said composition on a substrate. This step may comprise the step of
applying said ionomeric polymer as a tacky hot melt to the substrate;
controlling the operating temperature to avoid the decomposition temperature of any chlorite present; and
changing the viscosity and phase dispersion of the composition to control the reaction of the anhydride with the chlorite to release chlorine dioxide.

The method may further comprise the step of lowering the pH of said ionomeric polymer to less than 8.0.

The composition which liberates a repelling or biocidal agent when exposed to a water source may be said to comprise:
a cation;
a chlorite salt of said cation in a hydrogen bonded matrix; and
an incompatible phase having a hydrolyzable acid anhydride component therein mixed with said hydrogen bonded matrices to form an ionomeric composition having a chlorite compound contained therein such that said ionomeric composition releases chlorine dioxide when exposed to the water source for acting as a repelling or biocidal agent.

The chlorite salt may be derived of protonated primary amine, protonated secondary amine and protonated tertiary amine.

The cation may comprise an alkali, alkaline earth, transition metal, protonated primary amine, protonated secondary amine, protonated tertiary amine, quaternary monomeric amine, quaternary polymeric amine or a mixture thereof.

The ionomer composition may be meltable to create a film on a substrate.
In the above method and composition, the matrices may comprise aliphatic amides, aromatic amides, aliphatic alcohol, aromatic alcohol or a mixture thereof and may be selected from formamide, acrylamide-isopropylacrylamide mixtures or their polymerized products, neat primary or secondary amines and their polymerization products with acrylamide, isopropylacrylamide and N,N methylene bisacrylamide, and glycerine, ethylene glycol, or poly hydroxylic alcohols or mixtures of the above.

The incompatible phase with the hydrogen bonded phases may consist of grafted anhydride styrene copolymer of a concentration between approximately 5 weight percent and approximately 50 weight percent blended with or grafted to hydrocarbons such as polypropylenes, polyethylenes, or polystyrenes.

## Claims

1. A composition for disinfection, reducing bacterial and fungal growth, and repelling insects comprising:
a first phase containing a chlorite salt dissolved in a hydrogen-bonded matrix; and
an apolar second phase containing an anhydride and a diluent, the first and second phases being in intimate contact and incompatible with one another, and the first phase being capable of releasing chlorine dioxide upon hydrolysis of the anhydride.

2. A composition according to claim 1, wherein the first phase contains an amide, an alcohol, or a polymerizable hydrogen-bonded epoxide or amine.

3. A composition for disinfection, reducing bacterial and fungal growth, and repelling insects comprising:
a first phase containing a chlorite salt dissolved in a hydrogen-bonded matrix selected from an amide, an alcohol, or a polymerizable hydrogen-bonded epoxide or amine; and
an apolar second phase containing an anhydride, the first and second phases being in intimate contact and incompatible with one another and the first phase being capable of releasing chlorine dioxide upon hydrolysis of the anhydride.

4. A composition according to claim 2 or claim 3, wherein the amide of the first phase is selected from formamide, acrylamide-isopropylacrylamide, a copolymer of formamide and acrylamide-isopropylacrylamide, and a copolymer of acrylamide, isopropylacrylamide or N,N-methylene bisacrylamide and a primary amine or a secondary amine.

5. A composition according to claim 2 or claim 3, wherein the alcohol of the first phase is selected from methanol, glycerine, ethylene glycol, or ethoxyethanol.

6. A composition according to any one of claims 1 to 5, wherein the chlorite salt is derived from a protonated primary amine, a protonated secondary amine, a protonated tertiary amine, a quaternary amine, an alkali metal, an alkaline earth metal, or a transition metal.

7. A composition according to any one of claims 1 to 6, wherein the anhydride is blended with or grafted to polypropylene, polyethylene or polystyrene.

8. A composition according to any one of claims 1 to 7, wherein the anhydride is a copolymer of maleic anhydride and styrene.

9. A composition according to any one of claims 1 to 8, wherein the second phase contains atactic polypropylene, ethylbenzene, or an olefin.

10. A method of disinfecting, reducing bacterial and fungal growth, repelling insects from and/or deodorizing a substrate comprising:
exposing a substrate to a composition according to any one of claims 1 to 9; and
exposing the substrate to moisture to release chlorine dioxide from the composition into the atmosphere surrounding the substrate.

11. A method according to claim 10, wherein the substrate is coated with the composition and the coated substrate is exposed to moisture to release chlorine dioxide.

12. A method according to claim 11, wherein the substrate is coated with a film comprised of the composition.

13. A process for preparing a composition comprising:
dissolving a chlorite salt in a first material containing a hydrogen-bonded matrix to form a first phase; and
then mixing the first phase with an apolar phase containing an anhydride and a diluent, the first and second phases being incompatible with each other, the first phase being capable of releasing chlorine dioxide upon hydrolysis of the anhydride.

14. A process according to claim 13, wherein the first phase contains an amide, an alcohol, or a polymerizable hydrogen-bonded epoxide or amine.

15. A process for preparing a composition comprising:
dissolving a chlorite salt in a first material containing a hydrogen-bonded matrix to form a first phase, the hydrogen-bonded matrix being selected from an amide, an alcohol, or a polymerizable hydrogen-bonded epoxide or amine; and
then mixing the first phase with an apolar second phase containing an anhydride, the first and second phases being incompatible with each other, the first phase being capable of releasing chlorine dioxide upon hydrolysis of the anhydride.

16. A process according to any one of claims 13 to 15, wherein the second phase includes a diluent selected from the group consisting of atactic polypropylene, ethylbenzene, and an olefin.

17. A process according to any one of claims 13 to 16, further including the step of exposing the composition to moisture to hydrolyze the anhydride and release chlorine dioxide from the composition.

18. A process according to anyone of claims 13 to 17, further including the step of applying the composition to a substrate to form a film.

19. A process according to claim 18, wherein the composition is applied to the substrate at a temperature below that at which chlorite within the first material will decompose.

20. A process according to any one of claims 13 to 19, further including the step of changing the viscosity or dispersibility of the first and second phases, or changing the temperature of the composition to control release of chlorine dioxide from the composition once it is exposed to moisture.

21. A process according to any one of claims 13 to 20, wherein the chlorite salt is derived from a protonated primary, secondary, tertiary or quaternary amine, an alkali metal, an alkaline earth metal, or a transition metal.

## Patentansprüche

1. Zusammensetzung zur Desinfektion, Reduzierung des Bakterien- und Pilzwachstums und Abwehr von Insekten mit
einer ersten Phase, die ein in einer wasserstoffgebundenen Matrix gelöstes Chloritsalz enthält, und
einer ein Anhydrid und ein Verdünnungsmittel enthaltenden, apolaren zweiten Phase, wobei die erste und zweite Phase untereinander in innigem Kontakt und unverträglich sind und die erste Phase bei Hydrolyse des Anhydrids zur Freisetzung von Chlordioxid befähigt ist.

2. Zusammensetzung nach Anspruch 1, bei der die erste Phase ein Amid, einen Alkohol oder ein polymerisierfähiges, wasserstoffgebundenes Epoxid oder Amin enthält.

3. Zusammensetzung zur Desinfektion, Reduzierung des Bakterien- und Pilzwachstums und Abwehr von Insekten mit
einer ersten Phase, die ein Chloritsalz enthält, das in einer wasserstoffgebundenen Matrix gelöst ist, die unter einem Amid, einem Alkohol oder einem polymerisierfähigen, wasserstoffgebundenen Epoxid oder Amin ausgewählt ist, und
einer ein Anhydrid enthaltenden, apolaren, zweiten Phase, wobei die erste und zweite Phase untereinander in innigem Kontakt und unverträglich sind und die erste Phase bei Hydrolyse des Anhydrids zur Freisetzung von Chlordioxid befähigt ist.

4. Zusammensetzung nach Anspruch 2 oder Anspruch 3, bei der das Amid der ersten Phase unter Formamid, Acrylamid-Isopropylacrylamid, einem Copolymer aus Formamid und Acrylamid-Isopropylacrylamid und einem Copolymer aus Acrylamid, Isopropylacrylamid oder N,N-Methyllenbisacrylamid und einem primären Amin oder einem sekundären Amin ausgewählt ist.

5. Zusammensetzung nach Anspruch 2 oder Anspruch 3, bei der der Alkohol der ersten Phase unter Methanol, Glycerin, Ethylenglykol oder Ethoxyethanol ausgewählt ist.

6. Zusammensetzung nach einem der Ansprüche 1 bis 5, bei der das Chloritsalz von einem protoniertem primären Amin, einem protoniertem sekundärem Amin, einem protoniertem tertiären Amin, einem quaternären Amin, einem Alkalimetall, einem Erdalkalimetall oder einem Übergangsmetall abgeleitet ist.

7. Zusammensetzung nach einem der Ansprüche 1 bis 6, bei der das Anhydrid mit Polypropylen, Polyethylen oder Polystyrol gemischt oder auf sie aufgepfropft ist.

8. Zusammensetzung nach einem der Ansprüche 1 bis 7, bei der das Anhydrid ein Copolymer aus Maleinsäureanhydrid und Styrol ist.

9. Zusammensetzung nach einem der Ansprüche 1 bis 8, bei der die zweite Phase ataktisches Polypropylen, Ethylbenzol oder ein Olefin enthält.

10. Verfahren zum Desinfizieren, Reduzieren des Bakterien- und Pilzwachstums, Abwehren von Insekten und/oder Desodorieren von einem Substrat, bei dem man
ein Substrat einer Zusammensetzung nach einem der Ansprüche 1 bis 9 aussetzt und
das Substrat der Feuchtigkeit aussetzt, um Chlordioxid aus der Zusammensetzung in die das Substrat umgebende Atmosphäre freizusetzen

11. Verfahren nach Anspruch 10, bei dem das Substrat mit der Zusammensetzung beschichtet wird und das beschichtete Substrat zur Freisetzung von Chlordioxid der Feuchtigkeit ausgesetzt wird.

12. Verfahren nach Anspruch 11, bei dem das Substrat mit einem aus der Zusammensetzung bestehenden Film beschichtet wird.

13. Verfahren zur Herstellung einer Zusammensetzung, bei dem man
ein Chloritsalz in einem eine wasserstoffgebundene Matrix enthaltenden ersten Material unter Bildung einer ersten Phase auflöst und
dann die erste Phase mit einer ein Anhydrid und ein Verdünnungsmittel enthaltenden, apolaren Phase mischt, wobei die erste und zweite Phase untereinander unverträglich sind und die erste Phase bei Hydrolyse des Anhydrids zur Freisetzung von Chlordioxid befähigt ist.

14. Verfahren nach Anspruch 13, bei dem die erste Phase ein Amid, einen Alkohol oder ein Polymerisierbares, wasserstoffgebundenes Epoxid oder Amin enthält.

15. Verfahren zur Herstellung einer Zusammensetzung, bei dem man
ein Chloritsalz in einem eine wasserstoffgebundene Matrix enthaltenden, ersten Materialunter Bildung einer ersten Phase auflöst, wobei die wasserstoffgebundene Matrix aus einem Amid, einem Alkohol oder einem polymerisierbaren, wasserstoffgebundenen Epoxid oder Amin ausgewählt wird, und
dann die erste Phase mit einer ein Anhydrid enthaltenden, apolaren zweiten Phase mischt, wobei die erste und zweite Phase miteinander unverträglich sind und die erste Phase bei Hydrolyse des Anhydrids zur Freisetzung von Chlordioxid befähigt ist.

16. Verfahren nach einem der Ansprüche 13 bis 15, bei dem die zweite Phase ein Verdünnungsmittel enthält, das aus der aus ataktischem Polypropylen, Ethylbenzol und einem Olefin bestehenden Gruppe ausgewählt ist.

17. Verfahren nach einem der Ansprüche 13 bis 16, bei dem man ferner die Zusammensetzung der Feuchtigkeit aussetzt, um das Anhydrid zu hydrolysieren und das Chlordioxid aus der Zusammensetzung freizusetzen.

18. Verfahren nach einem der Ansprüche 13 bis 17, bei dem man ferner die Zusammensetzung auf ein Substrat aufbringt, um einen Film zu bilden.

19. Verfahren nach Anspruch 18, bei dem man die Zusammensetzung auf das Substrat bei einer Temperatur unterhalb von der aufbringt, bei der sich Chlorit in dem ersten Material zersetzt.

20. Verfahren nach einem der Ansprüche 13 bis 19, bei dem man ferner die Viskosität oder die Dispergierbarkeit der ersten und zweiten Phase verändert oder die Temperatur der Zusammensetzung verändert, um die Freisetzung des Chlordioxids aus der Zusammensetzung zu steuern, sobald diese der Feuchtigkeit ausgesetzt wird.

21. Verfahren nach einem der Ansprüche 13 bis 20, bei dem das Chloritsalz von einem protonierten primären, sekundären, tertiären oder quaternären Amin, einem Alkalimetall, einem Erdalkalimetall oder einem Übergangsmetall abgeleitet ist.

## Revendications

1. Composition destinée à la désinfection, diminuant la croissance bactérienne et fongique et repoussant les insectes, comprenant :
une première phase contenant un sel chlorite dissous dans une matrice à laquelle est lié de l'hydrogène ; et
une deuxième phase apolaire contenant un anhydride et un diluant, la première et la deuxième phase étant en contact intime et mutuellement incompatibles, et la première phase étant capable de libérer du dioxyde de chlore lors de l'hydrolyse de l'anhydride.

2. Composition selon la revendication 1, où la première phase contient un amide, un alcool ou un époxyde ou une amine polymérisables auxquels est lié de l'hydrogène.

3. Composition destinée à la désinfection, diminuant la croissance bactérienne et fongique, et repoussant les insectes, comprenant :
une première phase comprenant un sel chlorite dissous dans une matrice à laquelle est lié de l'hydrogène, sélectionnée parmi un amide, un alcool ou un époxyde ou une amine, polymérisables auxquels est lié de l'hydrogène ; et
une deuxième phase apolaire contenant un anhydride, la première et la deuxième phase étant en contact intime et mutuellement incompatibles et la première phase étant capable de libérer du dioxyde de chlore lors de l'hydrolyse de l'anhydride.

4. Composition selon la revendication 2 ou la revendication 3, où l'amide de la première phase est sélectionné parmi le formamide, l'acrylamide-isopropylacrylamide, un copolymère de formamide et d'acrylamide-isopropylacrylamide et un copolymère d'acrylamide, isopropylacrylamide ou N,N-méthylène-bisacrylamide et une amine primaire ou une amine secondaire.

5. Composition selon la revendication 2 ou la revendication 3, où l'alcool de la première phase est sélectionné parmi le méthanol, le glycérol, l'éthylèneglycol ou l'éthoxyéthanol.

6. Composition selon l'une quelconque des revendications 1 à 5, où le sel chlorite est dérivé d'une amine primaire protonée, d'une amine secondaire protonée, d'une amine tertiaire protonée, d'une amine quaternaire, d'un métal alcalin, d'un métal alcalino-terreux ou d'un métal de transition.

7. Composition selon l'une quelconque des revendications 1 à 6, où l'anhydride est mélangé avec du polypropylène, du polyéthylène ou du polystyrène ou greffé sur ces derniers.

8. Composition selon l'une quelconque des revendications 1 à 7, où l'anhydride est un copolymère d'anhydride maléique et de styrène.

9. Composition selon l'une quelconque des revendications 1 à 8, où la deuxième phase contient du polypropylène atactique, de l'éthylbenzène ou une oléfine.

10. Procédé de désinfection, diminuant la croissance bactérienne et fongique, repoussant les insectes d'un substrat et/ou désodorisant un substrat, comprenant :
l'exposition d'un substrat à une composition selon l'une quelconque des revendications 1 à 9 ; et
l'exposition du substrat à l'humidité pour libérer le dioxyde de chlore de la composition dans l'atmosphère entourant le substrat.

11. Procédé selon la revendication 10, où le substrat est revêtu de la composition et le substrat revêtu est exposé à l'humidité en vue de la libération du dioxyde de chlore.

12. Procédé selon la revendication 11, où le substrat est revêtu d'un film comprenant la composition.

13. Procédé de préparation d'une composition, comprenant :
la dissolution d'un sel chlorite dans une première substance contenant une matrice liée à l'hydrogène pour former une première phase ; et
le mélange subséquent de la première phase avec une phase apolaire contenant un anhydride et un diluant, la première et la deuxième phase étant mutuellement incompatibles, la première phase étant capable de libérer du dioxyde de chlore lors de l'hydrolyse de l'anhydride.

14. Procédé selon la revendication 13, où la première phase contient un amide, un alcool ou un époxyde ou une amine polymérisables auxquels est lié de l'hydrogène.

15. Procédé de préparation d'une composition, comprenant :
la dissolution d'un sel chlorite dans une première substance contenant une matrice à laquelle est liée de l'hydrogène pour former une première phase, la matrice à laquelle est lié de l'hydrogène étant sélectionée parmi un amide, un alcool ou un époxyde ou une amine polymérisables auxquels est lié de l'hydrogène ; et
le mélange subséquent de la première phase avec une deuxième phase apolaire contenant un anhydride, la première et la deuxième phase étant mutuellement incompatibles, la première phase étant capable de libérer du dioxyde de chlore lors de l'hydrolyse de l'anhydride.

16. Procédé selon l'une quelconque des revendications 13 à 15, où la deuxième phase comprend un diluant sélectionné dans le groupe constitué par le polypropylène atactique, l'éthylbenzène et une oléfine.

17. Procédé selon l'une quelconque des revendications 13 à 16, comprenant en outre l'étape consistant à exposer la composition à l'humidité pour hydrolyser l'anhydride et libérer le dioxyde de chlore de la composition.

18. Procédé selon l'une quelconque des revendications 13 à 17, comprenant en outre l'étape consistant à appliquer la composition sur un substrat pour former un film.

19. Procédé selon la revendication 18, où la composition est appliquée sur le substrat à une température inférieure à celle à laquelle le chlorite se trouvant dans la première substance va se décomposer.

20. Procédé selon l'une quelconque des revendications 13 à 19, comprenant en outre l'étape consistant à modifier la viscosité ou la dispersibilité de la première et de la deuxième phase ou à modifier la température de la composition pour maîtriser la libération du dioxyde de chlore de la composition, une fois exposée à l'humidité.

21. Procédé selon l'une quelconque des revendications 13 à 20, où le sel chlorite est dérivé d'une amine primaire, secondaire, tertiaire ou quaternaire protonée, d'un métal alcalin, d'un métal alcalino-terreux ou d'un métal de transition.
